# FASCICULE DE BREVET EUROPEEN

(11) **EP 3 993 748 B1**
(45) Date de publication et mention de la délivrance du brevet: **22.11.2023**
(21) Numéro de dépôt: 20735589.2
(22) Date de dépôt: 01.07.2020
(51) Int. Cl.: A61F 13/02

(54) **PANSEMENT AVEC DISTRIBUTION DES FLUIDES AMELIOREE**
VERBAND MIT VERBESSERTER FLÜSSIGKEITSVERTEILUNG
DRESSING WITH IMPROVED FLUID DISTRIBUTION

(30) Priorité: 02.07.2019 FR 1907343
(43) Date de publication de la demande: 11.05.2022
(73) Titulaire: Advanced Silicone Coating, 69330 Pusignan (FR)
(72) Inventeur: LECOEUVRE, Jean-François, 42290 SORBIERS (FR)
(74) Mandataire: Oudin, Stéphane
(86) Numéro de dépôt international: PCT/EP2020/068579
(87) Numéro de publication internationale: WO 2021/001454

(56) Documents cités:
- WO-A1-93/07841
- WO-A1-2009/081134
- WO-A1-2012/140377
- US-A- 5 632 731
- US-A1- 2010 292 626

## Description

### Domaine technique de l'invention

La présente invention se rapporte à un pansement absorbant destiné au traitement des plaies chronique ou aiguës, lequel pansement comprend un film imperméable aux fluides et perméable à la vapeur d'eau, une armature ajourée, une compresse absorbante et une couche de distribution des fluides insérée entre ledit film imperméable et ladite armature ajourée.

### État de la technique antérieure

Les pansements absorbants utilisés pour le traitement des plaies chroniques ou aiguës sont généralement constitués d'une compresse absorbante prise en sandwich entre un film de protection imperméable et respirant et une couche d'interface ouverte et enduite sur au moins une de ses faces d'un adhésif de silicone, pour éviter un contact direct entre la compresse et la plaie.

D'autres pansements comprennent une compresse absorbante destinée à être directement en contact avec la plaie. En effet, le contact direct entre la compresse et la plaie peut entraîner un effet thérapeutique bénéfique. Toutefois, en l'absence de l'interface ouverte enduite de silicone, ce type de pansement comprend un pourtour adhésif, appelé couramment trottoir, débordant de la compresse et permettant le maintien en place sur le patient.

Par exemple, la demande de brevet US 2010/0292626 décrit un pansement dans lequel seul le trottoir est revêtu d'adhésif silicone et la compresse absorbante est en contact direct avec la plaie. Pour la réalisation du pansement, un film de polyuréthane enduit d'adhésif de silicone est découpé à la forme du trottoir, puis collé à un film support imperméable et respirant au moyen d'un adhésif acrylique. La réalisation de ce type de pansement est complexe (étapes de découpe, centrage des composants) et entraîne une perte significative du matériau enduit d'adhésif de silicone pour réaliser la fenêtre accueillant la compresse et donner lieu au trottoir adhésif.

La demande WO2012140377 décrit un autre pansement comportant une compresse absorbante destinée au contact direct avec la plaie. Cette compresse est collée à un complexe imperméable, respirant et adhésif. Le support imperméable respirant et adhésif étant constitué de l'assemblage d'une armure textile tricoté ouverte, enduite sur ses deux faces d'adhésif de silicone contrecollée sur un film de polyuréthane imperméable et respirant. La présence de ce complexe permet d'améliorer la rigidité du pansement et facilite son utilisation. Par ailleurs, les portions de surface de film polyuréthane accessible au droit des porosités de l'armure textile présente une respirabilité importante. En revanche, la respirabilité est fortement limitée dans les zones pleines de l'armure textile. L'homme du métier doit donc trouver un compromis entre respirabilité et pouvoir adhésif, tous deux dépendant du rapport de la surface ouverte sur la surface totale (taux d'ouverture) avec des effets antinomiques. Ainsi, un fort taux d'ouverture favorisera une respirabilité élevée au détriment du pouvoir adhésif.

De façon alternative, l'armure textile ouverte enduite de gel adhésif de silicone peut être remplacée par un film thermoplastique, enduit d'adhésif sur ses deux faces, perforé et contrecollé à un film polyuréthane imperméable et respirant. Dans ce cas, pour un taux d'ouverture donné et une respirabilité donnée, il est possible d'améliorer plus aisément le pouvoir adhésif du pansement par le choix d'un adhésif de silicone à haute performances adhésive et par la quantité d'adhésif de silicone déposé. Il n'en reste pas moins nécessaire de trouver un compromis entre pouvoir adhésif et respirabilité.

Une autre alternative, décrite dans le brevet EP2231088B1, consiste à remplacer l'armure textile ouverte adhésive et le film adhésif double face par un filet unitaire thermoplastique enduit sur ses deux faces d'adhésif.

Toutefois, toutes ces alternatives présentent le même inconvénient qui est que toute variation du taux d'ouverture, que ce soit via une réduction des dimensions des trous, ou par la présence de trous mal ou pas débouché, se traduit par une forte baisse de respirabilité. Cette dernière peut nuire à la fonction de traitement ou soin de la plaie, voire induire une macération des tissus par excès d'humidité.

Par ailleurs, concevoir un pansement dont le niveau de respirabilité est très élevé, tout en garantissant la bonne adhésion de compresses lourdes une fois gavées d'exsudats, et notamment dans le cas des compresses dites « super-absorbantes » peut parfois se révéler impossible.

Il existe donc un besoin pour un pansement à compresse absorbante centrale qui soit imperméable aux liquides, qui présente un trottoir adhésif à hautes performances d'adhésion, une bonne adhésion de la compresse et une très forte respirabilité au droit de celle-ci. Par ailleurs, ce pansement doit-être aisé à fabriquer, sans pertes inutiles de support enduit d'adhésif de silicone.

### Résumé de l'invention

Ainsi la présente invention concerne notamment un pansement comprenant :
- un film imperméable aux fluides et perméable à la vapeur d'eau, recouvert sur l'ensemble de sa surface par,
- une armature ajourée enduite d'un adhésif médicalement acceptable, identique ou différent, sur chacune de ses faces, la face opposée à la face au contact dudit film imperméable étant partiellement recouverte par,
- une compresse absorbante,
remarquable en ce que ledit pansement comprend en outre une couche de distribution des fluides insérée entre ledit film imperméable et ladite armature ajourée.

Dans le cadre de la présente invention, le terme « être recouvert » entend désigner le fait qu'une première couche du pansement selon l'invention est en tout ou partie à l'aplomb d'une deuxième couche du pansement selon l'invention. Le terme recouvrir n'implique pas que lesdites couches soient en contact direct.

Dans le cadre de la présente invention, le terme « ajouré » entend désigner le fait que ladite armature n'est pas continue, mais présente des trous la traversant de part en part.

Dans le cadre de la présente invention, le terme « absorbant » entend désigner la capacité d'un matériau à retenir à l'intérieur de sa structure une certaine quantité d'eau. Selon un mode réalisation préféré de l'invention, un matériau est dit « absorbant » lorsqu'il est capable de retenir dans sa structure une masse de liquide supérieure à 1g pour 100cm² de matériau et encore plus préférentiellement 50g pour 100cm² de matériau (test fait selon la norme NF EN 13729-1 §3.2).

Dans le cadre de la présente invention, le terme « couche de distribution » entend désigner toute couche à l'intérieur de laquelle un liquide va pouvoir diffuser horizontalement et verticalement.

En effet, il a été trouvé de façon surprenante, que la présence d'une couche de distribution entre l'armature ajourée et le film imperméable permettait d'augmenter fortement la respirabilité de ce type de pansement par rapport aux pansements de l'art antérieur.

La couche de distribution permet le transfert des exsudats sur toute sa surface en contact avec le film. Dans un premier temps, les exsudats ne passent qu'au travers des porosités de l'armature ajourée, puis sont distribués horizontalement et verticalement grâce à l'action de la couche de distribution. Toute la surface du film imperméable aux fluides et perméable à la vapeur d'eau située au-dessus de la couche de distribution devient alors disponible pour laisser passer la vapeur d'eau. Il en résulte une très grande augmentation de la respirabilité du pansement dans cette zone. En conséquence, la respirabilité au droit de la compresse n'est pas limitée par le taux d'ouverture de l'armature ajourée collée au film imperméable aux fluides et perméable à la vapeur d'eau. Ainsi, il est possible d'utiliser des armatures ajourées au fort pouvoir adhésif pour obtenir un trottoir à hautes performances adhésives pour un bon maintien du pansement sur la peau du patient, et une haute performance adhésive au droit de la compresse permettant de garantir une bonne accroche de celle-ci, même une fois gavée d'exsudats.

Selon un mode de réalisation préféré de l'invention, ledit film imperméable aux fluides et perméable à la vapeur d'eau est en polyuréthane.

Selon un mode de réalisation préféré de l'invention, ladite armature ajourée est enduite sur sa face au contact dudit film imperméable d'un adhésif médicalement acceptable et sur l'autre de ses faces d'un gel de silicone adhésif.

Selon un mode de réalisation préféré de l'invention, ledit film imperméable aux fluides et perméable à la vapeur d'eau à une épaisseur comprise entre 5 µm et 100 µm et encore plus préférentiellement entre 10 µm et 40 µm.

Selon un mode de réalisation préféré de l'invention, ledit film imperméable aux fluides et perméable à la vapeur d'eau a une respirabilité MVTR à la vapeur d'eau en contact liquide, mesurée après 4 heures de tests selon la norme EN 13726-2:2002, comprise entre 500 g/m²/24h et 60 000 g/m²/24h et encore plus préférentiellement entre 10 000 g/m²/24h et 30 000 g/m²/24h.

Selon un mode de réalisation préféré de l'invention, ladite couche de distribution des fluides s'étend sur toute le surface de la compresse.

Selon un autre mode de réalisation préféré de l'invention, ladite couche de distribution des fluides s'étend sur une surface inférieure à celle de la compresse.

Selon un autre mode de réalisation préféré de l'invention, ladite couche de distribution des fluides s'étend sur toute la surface de la compresse et au-delà de la surface de la compresse.

Selon un mode de réalisation préféré de l'invention, ladite couche de distribution des fluides est composée d'un textile tissé ou tricoté, d'un non tissé ou d'un matériau absorbant.

Selon un mode de réalisation préféré de l'invention, ladite couche de distribution des fluides peut comprendre des additifs tels que des produits antimicrobien, anti odeur ou tout autres additifs supplémentaire.

Selon un mode de réalisation encore plus préféré de l'invention, ledit textile tissé ou tricoté est composé de fibres naturelles ou de fibres synthétiques

Selon un mode de réalisation encore plus préféré de l'invention, ledit textile non tissé est composé d'un matériau choisi dans le groupe comprenant le polyéthylène, polypropylène, polyester, polyamide et leurs mélanges.

Selon un mode de réalisation encore plus préféré de l'invention, ledit matériau absorbant est composé d'un matériau choisi dans le groupe consistant en le polyuréthane hydrophile, les fibres de carboxyméthylcellulose, le polyacrylate de sodium, l'alginate de sodium, le carboxyméthylcellulose, leurs dérivés et leurs mélanges.

Selon un mode de réalisation préféré de l'invention, ladite armature ajourée est un tricot ajouré, un filet unitaire en matériau thermoplastique, un film perforé ou un non tissé perforé.

Selon un mode de réalisation encore plus préféré de l'invention, ledit tricot ouvert est constituée de fibres synthétiques telles que polyéthylène, polypropylène, polyamide ou polyester, et encore plus préférentiellement de polyester.

Selon un mode de réalisation encore plus préféré de l'invention, ledit filet unitaire en matériau thermoplastique est constitué d'un matériau choisi dans le groupe consistant en le polyéthylène, polypropylène, polyamide, polyuréthane et leurs mélanges.

Selon un mode de réalisation encore plus préféré de l'invention, ledit film est constitué de polyuréthane ou de copolyester.

Selon un mode de réalisation préféré de l'invention, ledit adhésif est un hydrogel, un adhésif sensible à la pression (PSA) ou un adhésif à base de silicone et encore plus préférentiellement un gel de silicone adhésif.

Selon un mode de réalisation préféré de l'invention, le poids total d'adhésif sur l'ensemble des deux faces de l'armature ajourée est compris entre 30 g/m² et 500 g/m² et encore plus préférentiellement entre 100 g/m² et 300 g/m².

Selon un mode de réalisation préféré de l'invention, l'armature ajourée a un taux d'ouverture compris entre 5% et 50% et encore plus préférentiellement de 10 et 30%.

Selon un mode de réalisation préféré de l'invention, la compresse absorbante est constitué d'un matériau choisi dans le groupe consistant en les mousses hydrophiles de polyuréthane, les non tissés absorbants à base de fibres de carboxyméthylcellulose ou d'alginate de sodium, les composites à base de non tissé poreux comprenant en sandwich des matériaux absorbants et les sachets contenant des matériaux absorbants.

### Brève description des figures

[Fig. 1] présente une vue en coupe d'un mode de réalisation d'un pansement selon l'invention.
[Fig. 2] montre l'évolution de la respirabilité d'un mode de réalisation d'un pansement selon l'invention en fonction de la surface de la couche de distribution.
[Fig. 3] montre l'évolution de la respirabilité d'un mode de réalisation d'un pansement selon l'invention en fonction du taux d'ouverture de l'armature ajourée.

### Description détaillée de l'invention

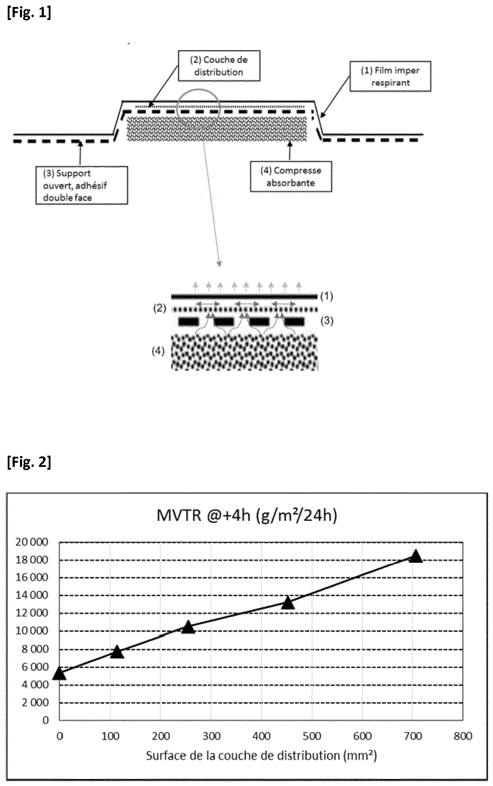

Le mode de réalisation préféré du pansement selon l'invention décrit à la figure 1 comprend :
- un film 1 imperméable aux fluides et perméable à la vapeur d'eau, recouvert sur l'ensemble de sa surface par,
- une armature ajourée 3 enduite sur sa face au contact dudit film imperméable d'un adhésif et sur l'autre de ses faces d'un gel de silicone adhésif, cette dernière face étant partiellement recouverte par,
- une compresse absorbante 4,
- une couche de distribution 2 des fluides insérée entre ledit film 1 imperméable et ladite armature ajourée 3.

La surface dudit pansement, sa forme, la surface du trottoir et de la compresse absorbante 4 est dépendante du type de plaies à traiter. L'homme du métier est à même de définir, pour chaque besoin, les surfaces optimums de chacun des composants.

La surface du trottoir peut être augmentée pour améliorer la capacité du pansement selon l'invention à adhérer sur la peau du patient.

L'adhésivité de l'armature ajourée 3 peut être également modifiée en faisant varier :
- la dimension des trous (longueur, largeur ou diamètre) qui peut varier entre 0.1 mm et 8 mm, de préférence entre 1.0 mm et 3 mm,
- leur répartition entre eux (e.g. positionnement à 45°, à 60° ou à 90°),
- leur densité en nombre par unité de surface, définie par un taux d'ouverture représenté par le ratio entre la surface ouverte rapportée à la surface totale.

Habituellement, la surface de la couche de distribution 2 est équivalente de celle de la compresse absorbante 4 placée au droit de celle-ci. Toutefois, ladite couche de distribution 2 peut avantageusement s'étendre au-delà de ladite compresse absorbante 4 afin d'améliorer la respirabilité du pansement au niveau du trottoir et d'évacuer l'humidité émise par sudation au niveau du trottoir.

Ladite couche de distribution 2 peut également présenter une surface inférieure à celle de la compresse 4 de façon à obtenir une respirabilité certes suffisante, mais pas excessive de façon à ne pas risquer d'assécher le lit de la plaie.

Les différentes couches du pansement selon l'invention peuvent être produites et assemblées par tout moyen connu de l'homme du métier.

Préférentiellement, le film imperméable aux fluides et perméable à la vapeur d'eau 1 peut être obtenu par extrusion gonflage, par extrusion à plat, par enduction liquide en phase solvant sur un support à base de papier ou de film synthétique l'un et l'autre revêtus d'une couche anti-adhérente.

Préférentiellement, l'armature ajourée 3 revêtue sur ses deux faces d'un ou de plusieurs adhésifs médicalement acceptables peut être obtenue par revêtement des deux faces, par exemple par enduction ou trempage dans un bain, d'un textile ouvert type tricot ajouré ou d'un filet unitaire en matériau thermoplastique, ou la perforation d'un film ou d'un non tissé préalablement enduit sur ses deux faces d'un ou de plusieurs adhésifs médicalement acceptables. Le tricot ouvert peut être obtenu par tricotage dit « chaîne ». Le filet unitaire en matériau peut être obtenu par extrusion casting puis étirage à chaud après perforation. Le film peut être obtenu par extrusion gonflage, par extrusion à plat, ou par enduction liquide en phase solvant sur un porteur. Le non tissé peut être obtenu par la technologie dite melt blown ou spunbond si les fibres sélectionnées sont thermoplastiques ou par technologie dite spunlace dans le cas de non tissés contenant des fibres non thermoplastiques.

Tous les matériaux cités dans la présente demande peuvent être avantageusement combinés à des agents fonctionnels pour le traitement de la plaie tels que des produits antimicrobien, désinfectant, anti odeur, activateur de soin de la plaie et/ou détersif.

Un traitement corona tel que décrit par la demanderesse dans le document WO 2015044535 A1 peut être avantageusement appliqué à l'adhésif de silicone sur les surfaces destinées à être collées à celui-ci (compresse 4 et/ou film imper respirant 1)

### Exemples

Exemple 1 : Mise en évidence de l'effet spécifique sur la respirabilité d'une couche de distribution 2.

Afin de démontrer l'effet de la couche de distribution 2 sur la respirabilité d'une construction selon l'invention, la déposante a construit des assemblages comprenant
-- un film imperméable aux fluides et perméable à la vapeur d'eau 1, recouvert sur l'ensemble de sa surface par,
   - une armature ajourée 3 enduite sur sa face au contact dudit film imperméable,
   - une couche de distribution des fluides 2 insérée entre ledit film imperméable et ladite armature ajourée (sauf échantillon témoin).

L'armature ajourée 3 était un film de polyuréthane, enduit sur une face d'adhésif acrylique et sur l'autre face d'adhésif de silicone, puis perforé et assemblé avec des films 1 de polyuréthane avec et sans (témoin) insertion d'une couche de distribution 2.

Dans l'assemblage obtenu, des disques assemblés de diamètre hors tout 42 mm ont été découpés, comprenant ou pas un disque de diffusion, collé en son centre, de diamètre arbitrairement défini à 24 mm, puis soumis au test de mesure de respirabilité en contact liquide (MVTR) selon la norme EN 13726-2:2002 pour une durée de 4h.

L'armature ajourée 3 a été fabriqué par la demanderesse sous la référence Acrysil 150 703731, avec des perforation trous à 60°, diamètre 2,4 mm et taux d'ouverture à 15%.

Les couches de distribution 2 évaluées sont :
- un non tissé polyester 45g/m² (référence Sontara Spunlace Style 8000 distribué par la société Jacob Holm Industries).
- une mousse de polyuréthane épaisseur 1.5mm (référence Vilmed 6217 fabriqué par la société Freudenberg).
- un non tissé absorbant (référence Vilmed M1556 fabriqué par la société Freudenberg).

Les films imperméables aux fluides et perméables à la vapeur d'eau 1 utilisés étaient:
- Un film d'épaisseur 15 µm (référence Inspire 2350, fabriqué par la société Transcontinental Advanced Coating Ltd).
- Un film d'épaisseur 30µm (référence Inspire 2301, fabriqué par la société Transcontinental Advanced Coating Ltd.).

La respirabilité MVTR observée pour chacune de ces constructions est indiquée dans le [Tableau 1] ci-dessous.

**[Tableau 1]**

| | Assemblage Témoin (sans couche de diffusion) | Assemblage avec non tissé polyester | Assemblage avec mousse de polyuréthane | Assemblage avec non tissé absorbant |
|---|---|---|---|---|
| Film PU 15µm | 5442 g/m²/24h | 14224 g/m²/24h | 14058 g/m²/24h | 20839 g/m²/24h |
| Film PU 30µm | 2945 g/m²/24h | 7664 g/m²/24h | 9443 g/m²/24h | 11448 g/m²/24h |

On observe ainsi, que de façon surprenante, le fait d'insérer une couche de diffusion 2 entre le l'armature ajourée 3 et le film 1, permet d'augmenter, de 160% à 290% selon les cas, la respirabilité du complexe.

De façon encore plus surprenante, la respirabilité augmente très fortement si la couche de distribution 2 tend à gonfler en absorbant le liquide (cas mousse Vilmed 6217), provoquant alors une élongation du film 1 de déformation puisque non collé ; il en résulte alors une plus grande respirabilité du film 1 par élongation, augmentation de surface d'échange et diminution d'épaisseur.

Exemple 2 : Mise en évidence de l'effet sur la respirabilité, d'un pansement selon l'invention, d'une couche de distribution 2.

Les assemblages de l'exemple 1 sont identiquement réalisés sur le film 1 de 15µm, mais un disque de mousse absorbante de polyuréthane est collé à l'armature ajourée 3, coté adhésif de silicone, pour figurer la compresse absorbante 4.

La compresse 4 en mousse absorbante de polyuréthane utilisé avait une épaisseur de 2 mm (référence MC F03 fabriquée par la société Advanced Medical Solutions Group Plc).

Les disques assemblés, de diamètre 42 mm, comprenant ou pas une couche de distribution 2 de diamètre 24 mm sont soumis au test de mesure de respirabilité en contact liquide (MVTR) selon la norme EN 13726-2:2002 pour une durée de 4h.

La respirabilité MVTR observée pour chacune de ces constructions est indiquée dans le [Tableau 2] ci-dessous.

**[Tableau 2]**

| | Assemblage Témoin (sans couche de diffusion) | Assemblage avec non tissé polyester | Assemblage avec mousse de polyuréthane | Assemblage avec non tissé absorbant |
|---|---|---|---|---|
| Film PU 15µm | 5414 g/m²/24h | 13767,6 g/m²/24h | 11964 g/m²/24h | 17136 g/m²/24h |

On constate ainsi que l'ajout d'une compresse absorbante 4 diminue légèrement la MVTR globale du pansement, mais l'effet dû à l'insertion de la couche de distribution 2 n'est pas aboli.

Exemple 3 : Effet de la variation de la surface de la couche de distribution 2 sur la respirabilité d'un pansement selon l'invention.

Des assemblages selon l'exemple 1 avec un film 1 de 15µm ont été réalisés en faisant varier le diamètre, donc la surface de la couche de distribution 2.

La couche de distribution 2 évaluée était un non-tissé polyester 45g/m² (référence Sontara Spunlace Style 8000 distribué par la société Jacob Holm Industries).

Les disques assemblés, de diamètre 42 mm, comprenant ou pas une couche de distribution 2 de diamètre variable (12, 18, 24 & 30 mm) ont été soumis au test de mesure de respirabilité en contact liquide (MVTR) selon la norme EN 13726-2:2002 pour une durée de 4h.

Les résultats obtenus sont présentés à la [Fig. 2].

On constate ainsi que la couche de distribution 2 remplit pleinement son rôle, l'humidité étant évaporée sur une plus grande surface de film 1.

En conséquence il est possible de faire varier la respirabilité MVTR en faisant simplement varier la surface de la couche de distribution 2. Dans certains cas, il peut être souhaitable d'avoir une respirabilité maximum pour éviter une accumulation d'exsudats au niveau de la plaie et ainsi une macération des tissus. Au contraire, dans certains cas, il convient de limiter la respirabilité du pansement pour éviter l'assèchement du lit de la plaie et ainsi favoriser la cicatrisation.

Exemple 4 : Effet de la variation du taux d'ouverture de l'armature ajourée 3 sur la respirabilité d'un pansement selon l'invention.

Des assemblages selon l'exemple 1, comprenant un film 1 de 15µm, ont été construits en faisant varier le design de perforation l'armature ajourée 3 de façon à générer des perforations ayant différents diamètres et taux d'ouverture.

Des assemblages, de diamètre 42 mm, comprenant ou pas un disque de distribution 3 de diamètre 24 mm sont soumis au test de mesure de respirabilité en contact liquide (MVTR) selon la norme EN 13726-2 :2002 pour une durée de 4h.

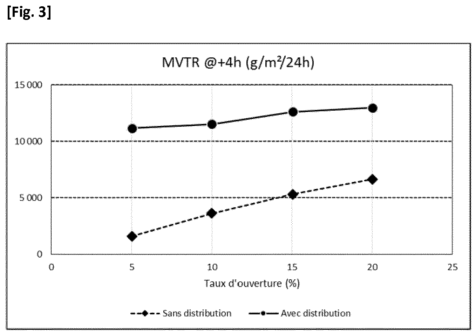

Les résultats obtenus sont présentés à la [Fig. 3].

On observe que, sans couche de distribution 2, la respirabilité reste fortement dépendante du taux d'ouverture l'armature ajourée 3. Inversement, en présence de la couche de distribution 2, la MVTR est quasiment stable en fonction du taux d'ouverture et donc du pouvoir adhésif du pansement selon l'invention.

En conséquence, contrairement au pansement de l'art antérieur, le pansement selon l'invention permet de modifier aisément la respirabilité du pansement sans modifier le taux d'ouverture du produit perforé et donc sans modifier son pouvoir adhésif sur le patient. Inversement, il est possible de moduler le pouvoir adhésif du pansement sur la peau et/ou de la compresse absorbante 4 sur l'armature ajourée 3 (en modifiant le taux d'ouverture de l'armature ajourée 3 et/ou la taille des trottoirs) sans modifier la respirabilité globale du pansement fini.

De façon complémentaire, il est ainsi possible de moduler et d'optimiser les deux caractéristiques de respirabilité et de pouvoir adhésif de façon totalement indépendante contrairement aux pansements de l'art antérieur.

## Revendications

1. Pansement comprenant :
-- un film (1) imperméable aux fluides et perméable à la vapeur d'eau, recouvert sur l'ensemble de sa surface par,
- une armature ajourée (3) enduite d'un adhésif médicalement acceptable, identique ou différent, sur chacune de ses faces, la face opposée à la face au contact dudit film (1) imperméable étant partiellement recouverte par,
- une compresse absorbante (4),
**caractérisé en ce que** ledit pansement comprend en outre une couche de distribution (2) des fluides insérée entre ledit film (1) imperméable et ladite armature ajourée (3).

2. Pansement selon la revendication précédente **caractérisé en ce que** ledit film (1) imperméable aux fluides et perméable à la vapeur d'eau est en polyuréthane.

3. Pansement selon l'une des revendications précédentes **caractérisé en ce que** ladite armature ajourée (3) est enduite sur sa face au contact dudit film (1) imperméable d'un adhésif médicalement acceptable et sur l'autre de ses faces d'un gel de silicone adhésif.

4. Pansement selon l'une des revendications précédentes **caractérisé en ce que** ledit film (1) imperméable aux fluides et perméable à la vapeur d'eau à une épaisseur comprise entre 5 µm et 100 µm.

5. Pansement selon l'une des revendications précédentes **caractérisé en ce que** ledit film (1) imperméable aux fluides et perméable à la vapeur d'eau a une respirabilité MVTR à la vapeur d'eau en contact liquide, mesurée après 4 heures de tests selon la norme EN 13726-2:2002, comprise entre 500 g/m²/24h et 60 000 g/m²/24h.

6. Pansement selon l'une des revendications précédentes **caractérisé en ce que** ladite couche de distribution (2) des fluides s'étend sur une surface inférieure à celle de la compresse absorbante (4).

7. Pansement selon l'une des revendications 1 à 5 **caractérisé en ce que** ladite couche de distribution (2) des fluides s'étend sur toute la surface de la compresse absorbante (4).

8. Pansement selon la revendication précédente **caractérisé en ce que** ladite couche de distribution des fluides (2) s'étend sur toute la surface de ladite compresse absorbante (4) et au-delà de la surface de la compresse absorbante (4).

9. Pansement selon l'une des revendications précédentes **caractérisé en ce que** ladite couche de distribution des fluides (2) est composée d'un textile tissé ou tricoté, d'un non tissé ou d'un matériau absorbant.

10. Pansement selon la revendication précédente **caractérisé en ce que** ledit textile tissé ou tricoté est composé de fibres naturelles ou de fibre synthétiques

11. Pansement selon la revendication 9 **caractérisé en ce que** ledit textile non tissé est composé d'un matériau choisi dans le groupe consistant en le polyéthylène, polypropylène, polyester ou polyamide et leurs mélanges.

12. Pansement selon la revendication 9 **caractérisé en ce que** ledit matériau absorbant est composé d'un matériau choisi dans le groupe consistant en le polyuréthane hydrophile, les fibres de carboxyméthylcellulose, le polyacrylate de sodium, l'alginate de sodium, le carboxyméthylcellulose et leurs mélanges.

13. Pansement selon l'une des revendications précédentes **caractérisé en ce que** ladite armature ajourée (3) est un tricot ajouré, un filet unitaire en matériau thermoplastique, un film perforé ou un non tissé perforé.

14. Pansement selon l'une des revendications précédentes **caractérisé en ce que** ledit adhésif est un hydrogel, un adhésif sensible à la pression (PSA) ou un adhésif à base de silicone.

15. Pansement selon l'une des revendications précédentes **caractérisé en ce que** le poids total d'adhésif sur l'ensemble des deux faces de l'armature ajourée (3) est comprise entre 30 g/m² et 500 g/m².

16. Pansement selon l'une des revendications précédentes **caractérisé en ce que** l'armature ajourée (3) a un taux d'ouverture compris entre 5% et 50%.

17. Pansement selon l'une des revendications précédentes **caractérisé en ce que** la compresse absorbante (4) est constituée d'un matériau choisi dans le groupe consistant en les mousses hydrophiles de polyuréthane, les non tissés absorbants à base de fibres de carboxyméthylcellulose ou d'alginate de sodium, les composites à base de non tissé poreux comprenant en sandwich des matériaux absorbants et les sachets contenant des matériaux absorbants.

## Patentansprüche

1. Verband, umfassend:
-- einen für Fluide undurchlässigen und für Wasserdampf durchlässigen Film (1), der auf der Gesamtheit seiner Fläche bedeckt ist von
- einer durchbrochenen Verstärkung (3), die auf jeder ihrer Seiten mit einem gleichen oder unterschiedlichen, medizinisch akzeptablen Kleber beschichtet ist, wobei die Seite, die der mit dem undurchlässigen Film (1) in Kontakt befindlichen Seite gegenüberliegt, teilweise bedeckt ist von
- einer absorbierenden Kompresse (4),
**dadurch gekennzeichnet, dass** der Verband weiter eine Schicht zum Verteilen (2) der Fluide umfasst, die zwischen dem undurchlässigen Film (1) und der durchbrochenen Verstärkung (3) eingefügt ist.

2. Verband nach dem vorstehenden Anspruch, **dadurch gekennzeichnet, dass** der für Fluide undurchlässige und für Wasserdampf durchlässige Film (1) aus Polyurethan besteht.

3. Verband nach einem der vorstehenden Ansprüche, **dadurch gekennzeichnet, dass** die durchbrochene Verstärkung (3) auf ihrer mit dem undurchlässigen Film (1) in Kontakt befindlichen Seite mit einem medizinisch akzeptablen Kleber, und auf der anderen ihrer Seiten mit einem klebenden Silikongel beschichtet ist.

4. Verband nach einem der vorstehenden Ansprüche, **dadurch gekennzeichnet, dass** der für Fluide undurchlässige und für Wasserdampf durchlässige Film (1) eine Dicke im Bereich zwischen 5 µm und 100 µm aufweist.

5. Verband nach einem der vorstehenden Ansprüche, **dadurch gekennzeichnet, dass** der für Fluide undurchlässige und für Wasserdampf durchlässige Film (1) eine Atmungsaktivität MVTR für Wasserdampf in Flüssigkeitskontakt, nach 4 Stunden Tests nach der Norm EN 13726-2:2002 gemessen, im Bereich zwischen 500 g/m²/24h und 60.000 g/m²/24h aufweist.

6. Verband nach einem der vorstehenden Ansprüche, **dadurch gekennzeichnet, dass** sich die Schicht zum Verteilen (2) der Fluide auf einer Fläche erstreckt, die kleiner ist als die der absorbierenden Kompresse (4).

7. Verband nach einem der Ansprüche 1 bis 5, **dadurch gekennzeichnet, dass** sich die Schicht zum Verteilen (2) der Fluide auf der ganzen Fläche der absorbierenden Kompresse (4) erstreckt.

8. Verband nach dem vorstehenden Anspruch, **dadurch gekennzeichnet, dass** sich die Schicht zum Verteilen der Fluide (2) auf der ganzen Fläche der absorbierenden Kompresse (4) und über die Fläche der absorbierenden Kompresse (4) hinaus erstreckt.

9. Verband nach einem der vorstehenden Ansprüche, **dadurch gekennzeichnet, dass** die Schicht zum Verteilen der Fluide (2) aus einem gewebten oder gestrickten Textil, aus einem Vlies oder aus einem absorbierenden Material zusammengesetzt ist.

10. Verband nach dem vorstehenden Anspruch, **dadurch gekennzeichnet, dass** das gewebte oder gestrickte Textil aus natürlichen Fasern oder aus synthetischen Fasern zusammengesetzt ist.

11. Verband nach Anspruch 9, **dadurch gekennzeichnet, dass** das Vliestextil aus einem Material zusammengesetzt ist, ausgewählt aus der Gruppe bestehend aus Polyethylen, Polypropylen, Polyester oder Polyamid und deren Mischungen.

12. Verband nach Anspruch 9, **dadurch gekennzeichnet, dass** das absorbierende Material aus einem Material zusammengesetzt ist, ausgewählt aus der Gruppe bestehend aus hydrophilem Polyurethan, Carboxymethylcellulose-Fasern, Natriumpolyacrylat, Natriumalginat, Carboxymethylcellulose und deren Mischungen.

13. Verband nach einem der vorstehenden Ansprüche, **dadurch gekennzeichnet, dass** es sich bei der durchbrochenen Verstärkung (3) um ein durchbrochenes Gestrick, ein einheitliches Netz aus thermoplastischem Material, einen perforierten Film oder ein perforiertes Vlies handelt.

14. Verband nach einem der vorstehenden Ansprüche, **dadurch gekennzeichnet, dass** es sich bei dem Kleber um ein Hydrogel, einen Haftkleber (PSA) oder einen Kleber auf Silikonbasis handelt.

15. Verband nach einem der vorstehenden Ansprüche, **dadurch gekennzeichnet, dass** das Kleber-Gesamtgewicht auf der Gesamtheit der zwei Seiten der durchbrochenen Verstärkung (3) im Bereich zwischen 30 g/m² und 500 g/m² beträgt.

16. Verband nach einem der vorstehenden Ansprüche, **dadurch gekennzeichnet, dass** die durchbrochene Verstärkung (3) eine Öffnungsquote im Bereich zwischen 5 % und 50 % aufweist.

17. Verband nach einem der vorstehenden Ansprüche, **dadurch gekennzeichnet, dass** die absorbierende Kompresse (4) aus einem Material besteht, ausgewählt aus der Gruppe bestehend aus hydrophilen Polyurethanschaumstoffen, absorbierenden Vliesen auf Basis von Carboxymethylcellulose- oder Natriumalginat-Fasern, Verbundstoffen auf Basis von porösem Vlies, die absorbierende Materialien in sandwichartiger Struktur umfassen, und Beuteln, die absorbierende Materialien enthalen.

## Claims

1. Dressing comprising:
-- a film (1) which is impermeable to fluids and permeable to water vapour, and the entire surface of which is covered by,
- a perforated reinforcement (3) coated with a medically acceptable adhesive which is the same or different on each of the faces thereof, the face opposite to the face in contact with said impermeable film (1) being partially covered by,
- an absorbent pad (4),
**characterised in that** said dressing further comprises a fluid distribution layer (2) inserted between said impermeable film (1) and said perforated reinforcement (3).

2. Dressing according to the preceding claim **characterised in that** said film (1) impermeable to fluids and permeable to water vapour is made of polyurethane.

3. Dressing according to one of the preceding claims **characterised in that** said perforated reinforcement (3) is coated on the face thereof in contact with said impermeable film (1) with a medically acceptable adhesive and on the other face thereof with an adhesive silicone gel.

4. Dressing according to one of the preceding claims **characterised in that** said film (1) impermeable to fluids and permeable to water vapour has a thickness between 5 µm and 100 µm.

5. Dressing according to one of the preceding claims **characterised in that** said film (1) impermeable to fluids and permeable to water vapour has an MVTR breathability to water vapour in liquid contact, measured after 4 hours of tests as per the EN 13726-2:2002 standard, between 500 g/m²/24h and 60,000 g/m²/24h.

6. Dressing according to one of the preceding claims **characterised in that** said fluid distribution layer (2) extends over a surface less than that of the absorbent pad (4).

7. Dressing according to one of claims 1 to 5 **characterised in that** said fluid distribution layer (2) extends over the entire surface of the absorbent pad (4).

8. Dressing according to the preceding claim **characterised in that** said fluid distribution layer (2) extends over the entire surface of said absorbent pad (4) and beyond the surface of the absorbent pad (4).

9. Dressing according to one of the preceding claims **characterised in that** said fluid distribution layer (2) is composed of a woven or knit textile, a nonwoven material, or an absorbent material.

10. Dressing according to the preceding claim **characterised in that** said woven or knit textile is composed of natural fibres or of synthetic fibres.

11. Dressing according to claim 9 **characterised in that** said nonwoven textile is composed of a material chosen in the group consisting of polyethylene, polypropylene, polyester or polyamide and mixtures thereof.

12. Dressing according to claim 9 **characterised in that** said absorbent material is composed of a material chosen in the group consisting of hydrophilic polyurethane, carboxymethylcellulose fibres, sodium polyacrylate, sodium alginate, carboxymethylcellulose, and mixtures thereof.

13. Dressing according to one of the preceding claims **characterised in that** said perforated reinforcement (3) is a perforated knit, a unitary net made of thermoplastic material, a perforated film or a perforated nonwoven.

14. Dressing according to one of the preceding claims **characterised in that** said adhesive is a hydrogel, a pressure-sensitive adhesive (PSA) or a silicone-based adhesive.

15. Dressing according to one of the preceding claims **characterised in that** the total weight of adhesive on all of both faces of the perforated reinforcement (3) is between 30 g/m² and 500 g/m².

16. Dressing according to one of the preceding claims **characterised in that** the perforated reinforcement (3) has an opening rate between 5% and 50%.

17. Dressing according to one of the preceding claims **characterised in that** the absorbent pad (4) consists of a material chosen in the group consisting of hydrophilic polyurethane foams, absorbent nonwoven materials based on carboxymethylcellulose fibres or sodium alginate, composites based on porous nonwoven materials comprising sandwiched absorbent materials and sachets containing absorbent materials.
